# EUROPEAN PATENT SPECIFICATION

(11) **EP 2 914 196 B1**
(45) Date of publication and mention of the grant of the patent: **04.12.2019**
(21) Application number: 13848143.7
(22) Date of filing: 25.07.2013
(51) Int. Cl.: A61B 17/06, A61B 17/42

(54) **MEDICAL INSTRUMENTS KIT FOR TREATING UTERUS PROLAPSE**
MEDIZINISCHES INSTRUMENTENKIT ZUR BEHANDLUNG EINES UTERUSPROLAPS
KIT D'INSTRUMENTS MÉDICAUX POUR TRAITER UN PROLAPSUS DE L'UTÉRUS

(30) Priority: 31.10.2012 RO 201200777
(43) Date of publication of application: 09.09.2015
(73) Proprietor: Nahedd, Saba, Jud. Ilfov (RO)
(72) Inventor: Nahedd, Saba, Jud. Ilfov (RO)
(74) Representative: Apostol, Salomia
(86) International application number: PCT/RO2013/000014
(87) International publication number: WO 2014/107117

(56) References cited:
- WO-A2-2004/016196
- WO-A2-2010/093421
- US-A1- 2008 114 382
- US-A1- 2008 287 956
- US-A1- 2011 065 992

## Description

The invention relates to a medical instruments kit for a surgical intervention with a view to treating the advanced anterior vaginal wall prolapse, very large cystocele, named "cystocele permagna" and the stage II and III uterus prolapse.

The uterus prolapse means the uterus descent into the vaginal axis and outside it, accompanied by the movement, to the same direction, of the vagina walls and of the adjacent portions of the urinary bladder and rectum. The descent is progressive into the small basin of the uterus, as a consequence of relaxation of the muscles and fibrous and extensible tissues of perineum - muscles forming the basin basis - as well as of the means of sustaining the organs of the small basin.

This explains the herniation, and the uterus prolapse, respectively, outside the vulvar commissure in various degrees, with the occurrence of the clinical symptoms consisting of heaviness in the lower portion of the pelvis, walking lumbar pain, urination and sexual intercourse pain, the sensation that "something is going to fall out/sits on a bali", the introitus irritation and vagina ulcerations.

The uterus prolapse particularly affects the post-menopausal women, the women who had a difficult natural childbith or several childbirths.

All these symptoms create a biological and social discomfort for the female patient.

The uterus prolapse is often associated to stress urinary incontinence, stress loss of some urine drops, sometimes masked by the excessive uterus prolabation.

It is worth mentioning that after any surgical treatment for correcting the uterus prolapse there must also be considered the correction of the' stress urinary incontinence which is sometimes masked.

There are known medical instruments kits for a surgical intervention which consists in suspending the uterus at the promontorium - hysteropexy or sacropexy - while opening the peritoneal cavity, said kits containing a bistoury, some scissors, some Pean clamps, some Kocher clamps, a metallic bladder probe, some Farabeuf spacing devices, non-absorbable suturing threads, and a propylene mesh, same Pereyra-type clamps and needles or a Reverdyn-type clamp.

*There are known medical instrument kits comprising a clampfor anchoring the uterine isthmus and some isthmic and sub urethral strips, respectively, the clamp consisting of two long rigid arms provided with some posterior portion spaced apart from each other, joined at the lower side by means of a bridge in from of which the distance between the arms has the minimum value, the latter being non-removable joined together and having a smooth blunt tip, in the immediate proximity thereof in a proximal curved zone of the anterior portion there being cut some upper and lower orifices, respectively.*

These kits have the disadvantages of not allowing to carry out, with full safety, an anatomical reconstruction surgical intervention without the occurrence of the pelvic pain syndrome, considered anatomically distorslonal. Since the Pereyra clamps and needles penetrate from the upper edge of the pubic symphysis, crossing the aponeurosis of the rectus abdominis and retropubic muscles, reaching the vagina, there exists a great probability of injuring and perforating the urinary bladder the more so as they can not be oriented during the use.

With regard to the construction and use of the Reverdyn-type clamp, this is onceived so as to be inserted through the retropubic tunnels from the bottom to the top, oriented psuprasynphysiarily to the aponeurosis of the rectus abdominis muscles, thus there existing a great probability of injuring and producing continuity solutions at the urinary bladder level, the more so as it can not be oriented during the use; this fact justifies the low surgical results, but always disputable, of the anatomical distorsion surgical intervention.

There are known methods for the treatment of the uterus prolapse, which comprise either a hysterectomy, which consists in extirpating the uterus through the vaginal route, or a hysteropexy, which consists in fixing the uterus to a stable element inside the small basin, by means of a thread or strips by fixing to a ligament of the first sacral *vertebra,* named promontofixation or by crossing the round ligaments which sustain the uterus, named ligamentary hysteropexy, either the anterior colporrhaphy and posterior colpoperineorrhaphy with the myorrhaphy of the annus raising muscles, which consists in reconstructing the muscles of the pelvic floor, in order to consolidate the pelvic organs support, or the triple Manchester operation consisting of the uterine cervix amputation, anterior colporrhaphy and posterior colpoperineorrhaphy.

The disadvantages of these methods consist in that either the vaginal vault prolapse occurs after some time, or they lead to the occurrence of a pelvic pain syndrome, having a relatively high percentage of recurrence,but none of the methods achieves the anatomical repositioning of the uters into its normal position, so as to eliminate the unavoidable urinary incontinence when the urinary bladder structures have been affected by removing the uterus, and the uterus prolapse in most cases is solved partially, with relatively high chances of the prolapse recurrence after a while.

There are also known methods for treating the urinary bladder prolapse, named cystocele, which consist in inserting a permanent implant consisting of a central monofilament mesh made of polypropylene, placed between four arms of the same material.

The disadvantages of these methods consist in that they only deal with the cystocele, with a relatively low success, and result in the occurrence of a pain syndrome and do not solve the uterus prolapse.

WO2010093421 A2 discloses devices, implants and kits for treating incontinence. In particular an assembly comprising a multi-piece implant comprising a support portion piece and two extension portion pieces adjustably connected to the support portion piece, and an adjusting tool to allow adjustment of extension portion pieces relative to the support portion piece, is described.

The technical problem solved by the kit according to the claimed invention consists in safely performing the uterine isthmus anchoring to the rectus abdominis muscles sheath, within a reconstructive surgical intervention, for the restoration of the anatomical uterus connections and anatomical repositioning of the uterus into its normal position.

According to the invention, the kit solves the technical problem and eliminates the previously shown disadvantages in that it also comprises a clamp for passing the isthmus anchoring threads and some isthmic and suburetral strips, the clamp consisting of two long rigid arms provided with some posterior portions spaced apartfrom one another, united to the lower side, by means of a bridge, in front of which the distance between the arms has the maximum value, said arms being non-removably joined to each other to form an anterior portion; tapered towards an anterior smooth blunt tip in the immediate proximity thereof, in a proximal curved zone of the anterior portion there being cut some upper and lower orifices, respectively, in front of the bridge the long arms being provided distally with two left side and right side rings, respectively, to a lower portion of the right side ring there being integrally attached an additional ring, to the external side of the long arms, in the proximity of the bridge and before the beginning of the anterior portion, there being placed two short left side and right side arms, respectively, said arms ending with some smooth blunt tips.

The kit claimed by the invention also solves the technical problem in that the clamp for anchoring the uterine isthmus has a length of 28 cm, and from the level of the additional ring the long arms are rectilinear, for a length of 22 cm and curved for a length of 6 cm, with an angle of 30° in a proximal curved zone.

The kit claimed by the invention also solves the technical problem in that the short arms are located at a distance of 4 cm away from the bridge and have a length of 2.5 cm and make an angle of 20° with the long arms.

The kit claimed by the invention also solves the technical problem in that at a distance of 1.0 cm away from the blunt tip of the proximal curved zone there are provided the upper and lower orifices, respectively, said orifices having a diameter of 3.0 ...4.0 mm, with a distance of 4.5 ... 5.5 mm therebetween.

The kit claimed by the invention also solves the technical problem in that the isthmic strip is made of a medically acceptable non-absorbable material, such as propylene and consists of two long and short portions, respectively, the short portion being integrally connected to the long portion in front of a medial zone thereof, each of some ends of the long portion being linked with one of these threads made of the same non-absorbable material, and an inner end of the short portion being free.

The kit claimed by the invention also solves the technical problem in that the mesh size of the non-absorbable material that the long and short portions are made of is of 0.03 mm, the lengths of the portions are of 16.0 cm and 8.0 cm, respectively, and their width is of 1.2 cm.

The kit claimed by the invention also solves the technical problem in that the suburethral strip consists of a body made of polypropylene, each of some ends thereof being ligated with one of some threads made of polypropylene, the body length being of 10 cm and the width thereof being of 1.2 cm.

The technical problem is also solved *by the use of* the medical instruments kit for reconstructing the adjacent anatomical connections for the degree II or degree III uterus prolapse, and for advanced cystocele permagna, respectively.

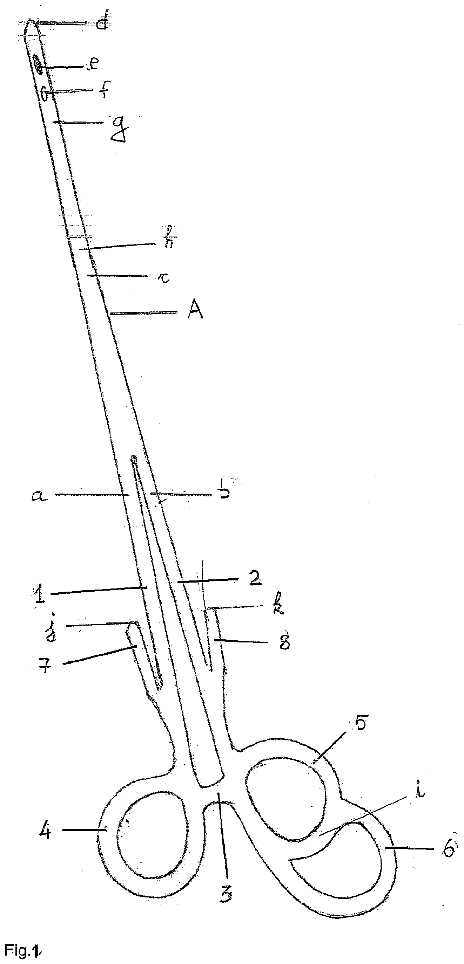

By applying the kit claimed by the invention the following advantages are obtained:
- the clamp is easy to be handled and allows the threads of both strips to pass at the same time through a tunnel;
- the use of non-absorbable polypropylene material for making the isthmic and suburethral strips and of the threads to be fixed to the rectus abdominis muscles sheath - a hormonally independent tissue
- makes the recurrence risks as low as possible or almost inexistent;
- there is solved the uterus prolapse and cystocele permagna and the uterus is brought back to its anatomical position;
   placing the uterus in the intermediary normal position avoids the extension of the retrovaginal space and consequntly prevents the occurrence of retrocele and elitrocele;
- there is solved the stress urinary incontinence;
- the surgical approach is exclussively on the vaginal route, thereby the incidents and accidents of opening the peritoneal cavity being avoided.
There are given hereinafter an embodiment for carrying out the kit as claimed by the inventions, in connection with Fig. 1... 14, which represent:
- Fig. 1 front view of a clamp for anchoring the uterine isthmus;

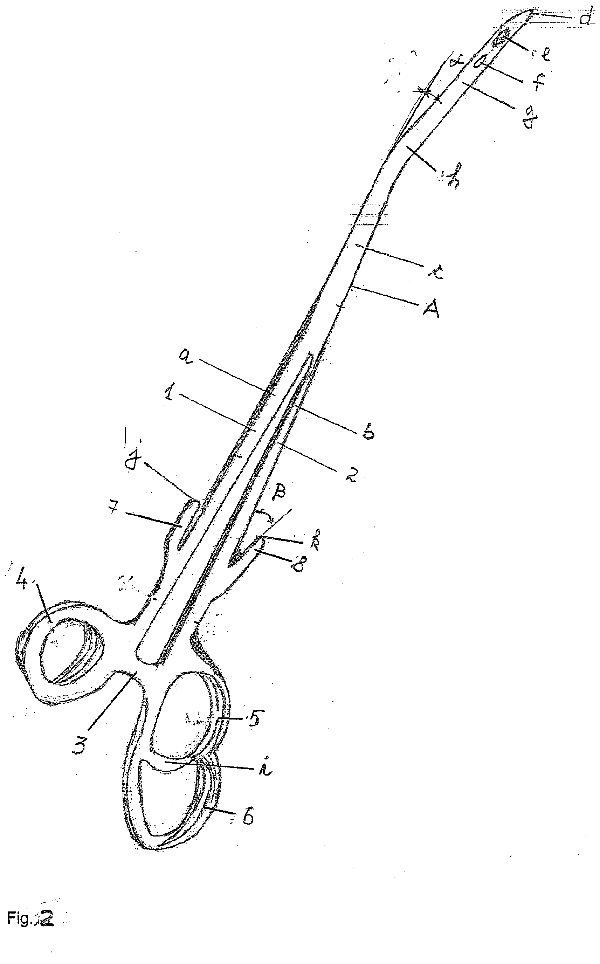

- Fig. 2 lateral perspective view of the clamp for anchoring the uterine isthmus;

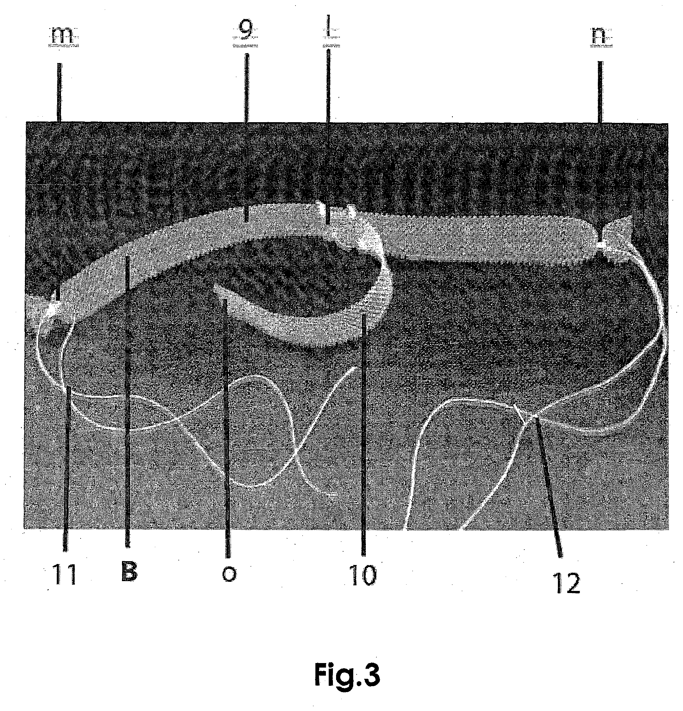

- Fig. 3 lateral perspective view of an isthmus strip;

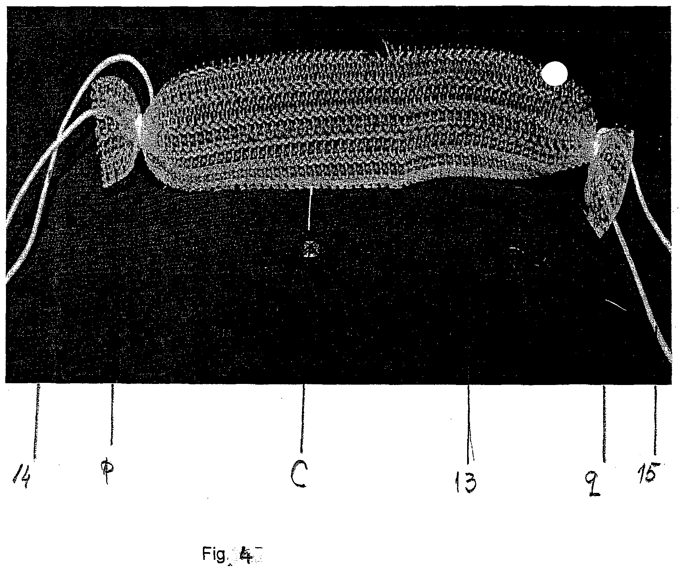

- Fig. 4 lateral perspective view of a suburethral strip;

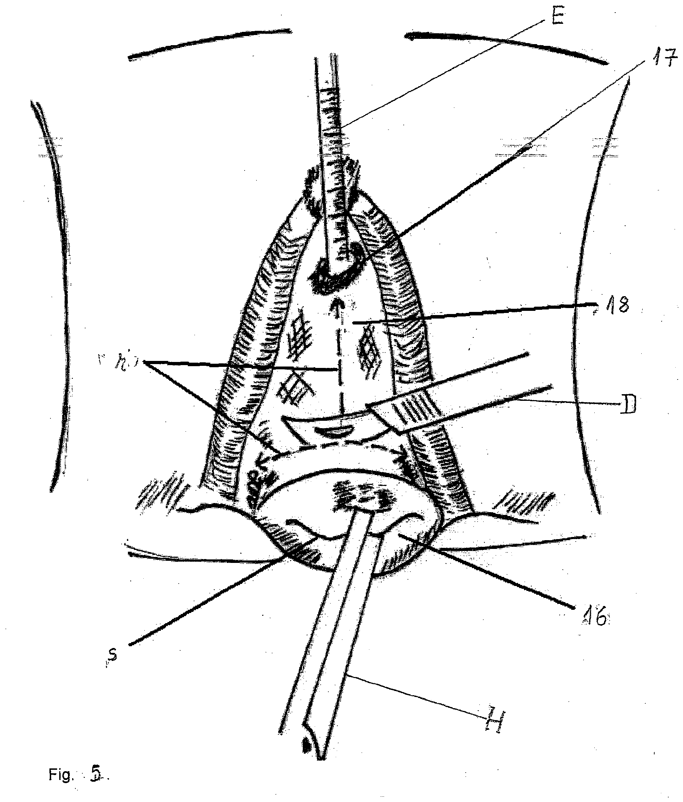

- Fig.5 schematic perspective view of carrying out, in time 1, a reversed T-shaped incision extending in the proximity of the external uterine orifice up to the urethral tubercle;

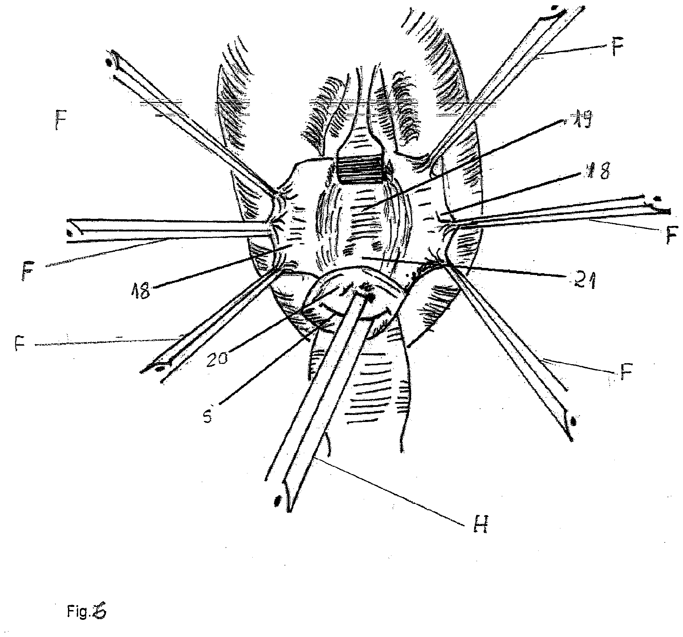

- Fig. 6 a schematic view of carrying aut, in time 2, the removal of the bladder from anterior vaginal wall and the bladder from the uterine cervix;

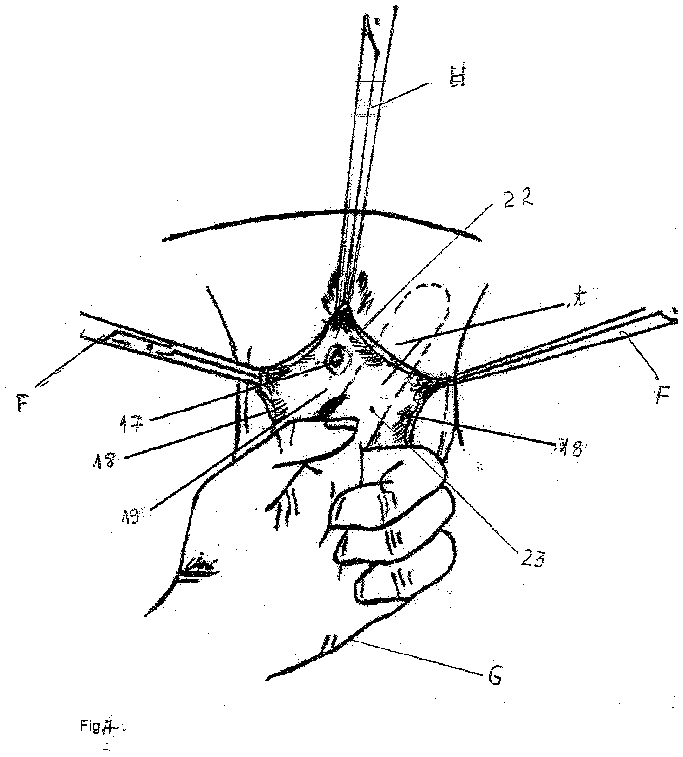

- Fig. 7 schematic view of carrying out, in time 3, some retropubic tunnels;

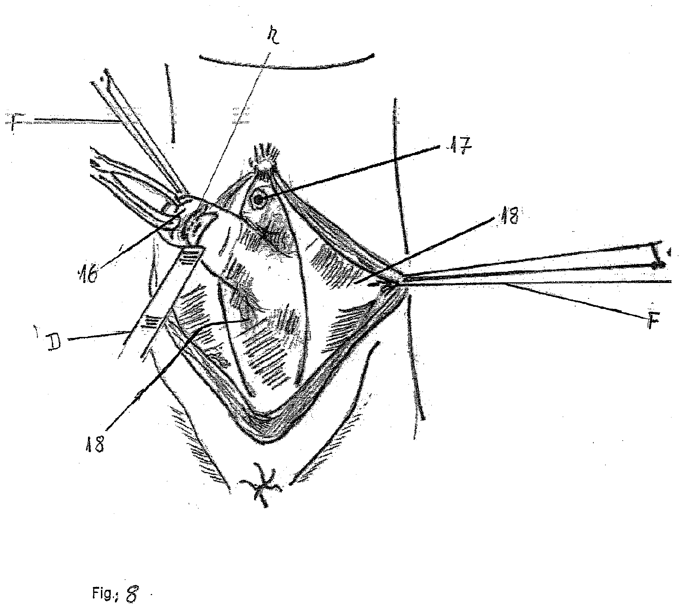

- Fig. 8 schematic view of continuing, in time 4, the incision on the posteror face of the uterine cervix;

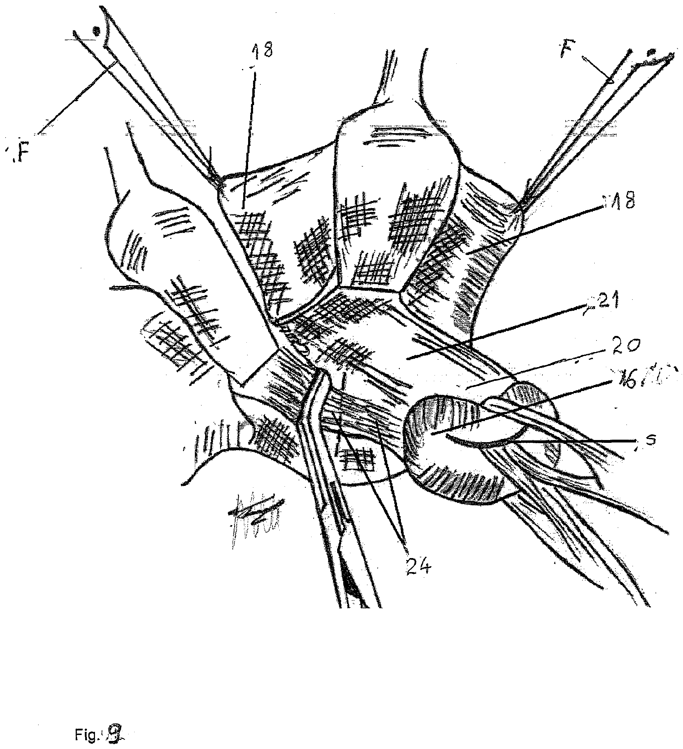

- Fig. 9 schematic view of carrying out, in time 5, clamping, sectioning and ligating the cardinal ligaments;

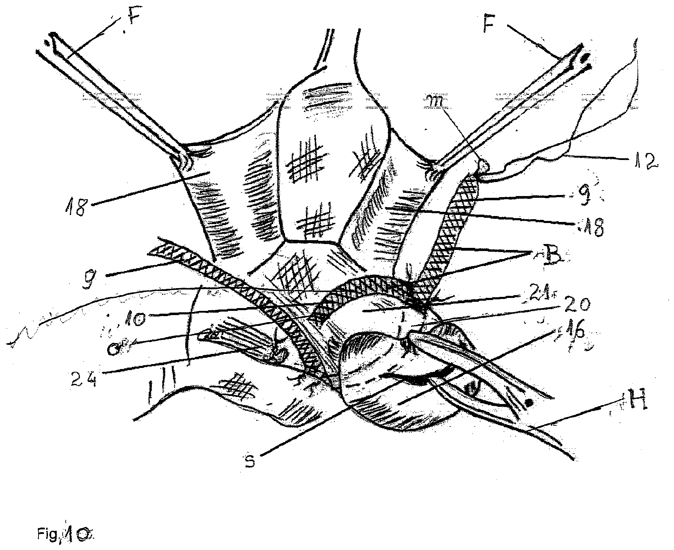

- Fig.10 schematic view of fixing, in time 6, a long isthmic strip onto the lateral and posterior faces of the uterine isthmus;

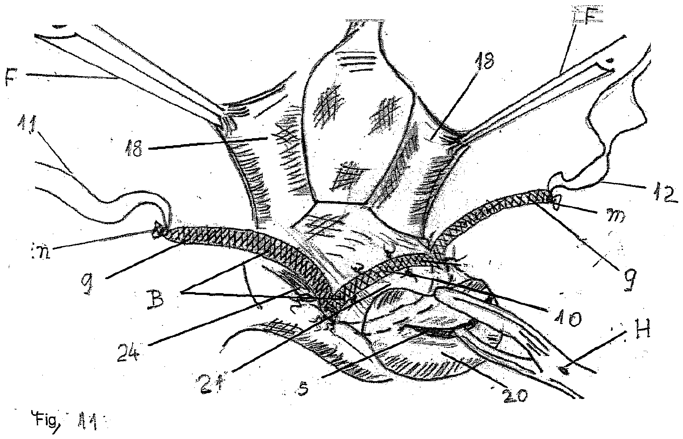

- Fig.11 schematic view of fixing, in time 7, the free short portion of the isthmic strip on the anterior face of the uterine isthmus;

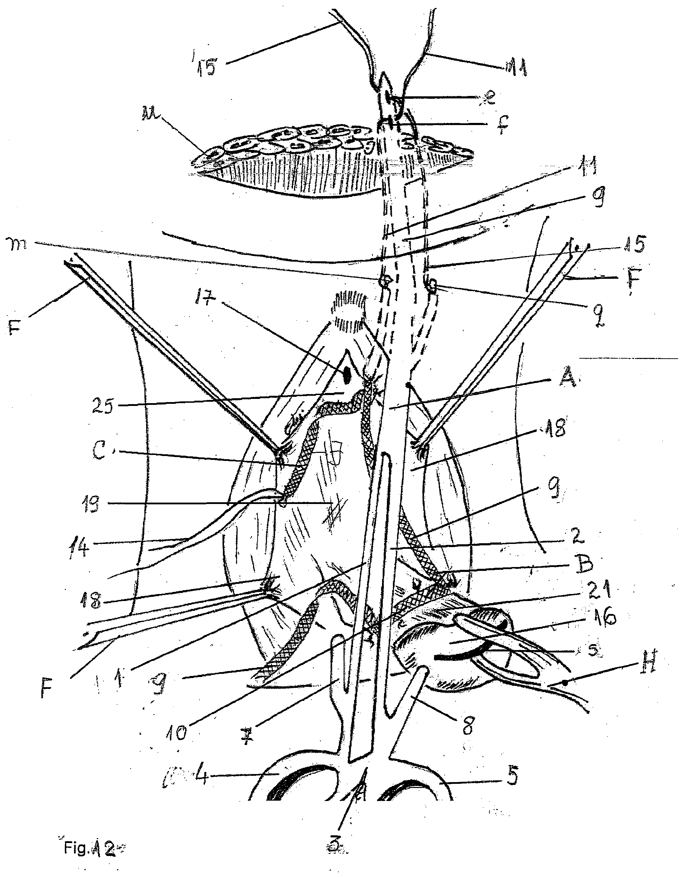

- Fig.12 schematic view of mounting, in time 8, by means .of the clamp, the ends of the two threads of some suburethral strips and some isthmic strips, respectively, through the two orifices of the clamp, and of passing the same through the tunnels;

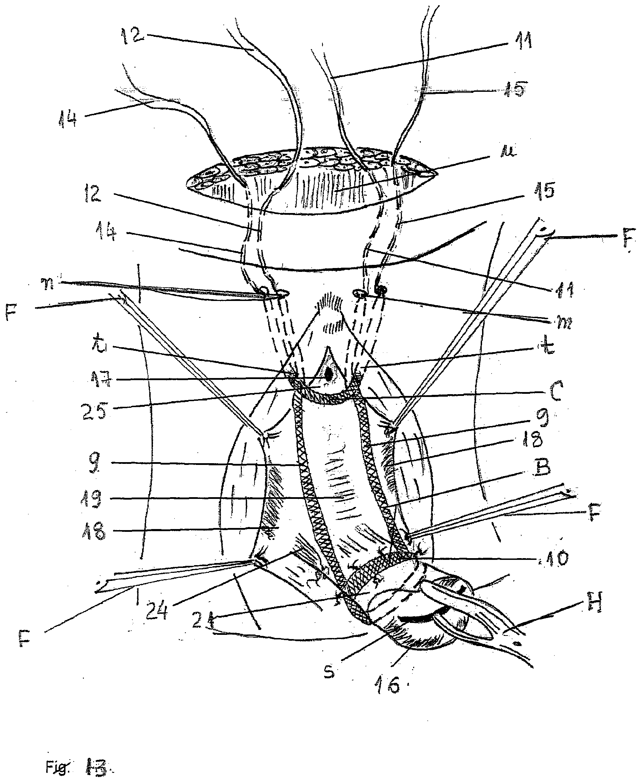

- Fig.13 schematic view of passing, in time 9, the ends of the suburethral strip to the external extremities of the suprapubic incision, and of the isthmic strip thread ends in the medial zone of the suprapubic incision;

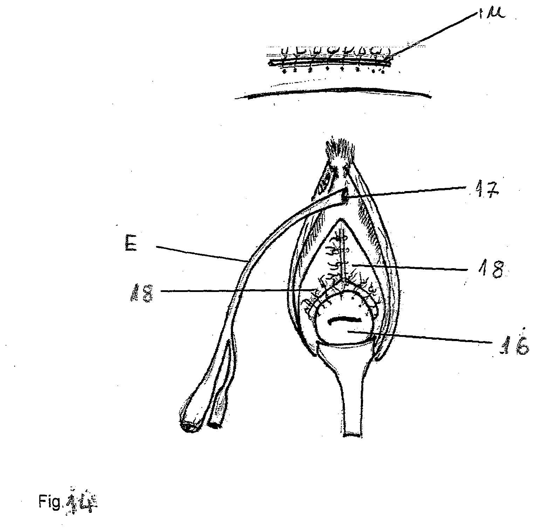

- Fig.14 schematic view of carrying out, in time 10, the suprapubic transverse incision suturing which represents the final image of the surgical interventionon.

The kit claimed by the invention comprises **a** box not represented in the figures, which is provided with some compartments for accommodating a bistoury, 6 Pean clamps, 3 Kocher clamps, two surgical scissors, absorbable and non-absorbable suturing threads, a clamp **A** for anchoring the uterine isthmus, an isthmic strip **B**, a strip **C** for urethrocystopexy, a metallic bladder probe and two Farabeuf spacing devices, of which only the clamp **A** and strips **B** and **C** are represented in the figures, the other medical instruments and the strip C being known per se.

The clamp **A** consists of two long rigid arms **1** and **2** provided with some posterior portions **a** and **b** spaced apart from each other, joined at the lower side by means of a bridge **3**, in front of which, between the arms **1** and **2** there is a distance which is reduced gradually until these make one piece.

The arms **1** and **2** are joined to each other by welding, forming an anterior portion c tapered towards an anterior smooth blunt tip **d**, the legth of which is of 2.5 cm.

At a distance of 10 cm away from the tip **d**, in the portion c there is provided an upper orifice e with a diameter of 3.0 ...4.0 mm, and at a distance of 4.5 ... 5.5 mm away from the centre of the orifice **c** there is provided a lower orifice f having the same diameter as the orifice **e.**

The portion **c** has a proximal curved zone **g** whose axis is inclined by an angle **α** of 30° in relation to the axis of a lower zone **h.**

The arms **1** and **2** are distally provided with two profiled left side and right side rings **4** and **5**, located in front of the bridge **3.** To a lower portion **i** of the ring **5** there is rigidly attached an additional ring **6.**

To the outer side of the arms **1** and **2**, at a distance of **4** cm away from the bridge **3**, there are placed two short left side and right side arms **7** and **8**, respectively, which make an angle *β* of 20° with the arms **1** and **2**, each of them having a length of 25 cm. each of the short arms **7** and **8** ending with one of some smooth blunt tips **j** and **k.**

The total length of the clamp **A** is preferably of 28 cm, and from the level of the additional ring **6**, the arms **1** and **2** are rectilinear on a length of 22 cm, then continuing with a proximal inclined zone **g** having a length of 6 cm.

The strip **B** is made up of a medically acceptable non-absorbable material which can be polypropylene and which has a long portion **9**, to which, in front of a medial zone **l** thereof, there is fixed a short portion **10.** The size of the non-absorbable material meshes is preferably of 0.03 mm, the lengths of the portions **9** and **10** are equal to 16.0 cm and 8.0 cm, respectively, and the width of the portions **9** and **10** is equal to 1.2 cm. Each of some ends m and n of the portion 9 are ligated with one of some non-absorbable threads **11** and **12**, and an interior end **O** of the portion 10 is free. The free end **O** of the portion 10 is cut during the surgical intervention, according to the patient's dimensions and it is the sutured below a long portion **9.** The free portion **10** starts from a distance of 5.0 cm from the end m of the long portion **9.**

The suburethral strip **C** consists of a body **13** made of the same medically acceptable non-absorbable material that the strip **B** is made of and has some ends **p** and **q**, each being ligated with one of some further non-absorbable threads **14** and **15.** The length of the body **13** is of **10** cm and the width thereof is of 1.2 cm.

In order to anchor the uterine isthmus to the rectus abdominis muscles sheath as close as possible to the normal anatomical location, during the, operating time **I** of a surgical operation, a reversed "T"-shaped incision **r** is made by means of the bistoury **D** starting from the level of the uterine cervix **16.**

The incision **r** is carried out transversally with a length of 2.0 cm, 1.5 cm above an external uterine orifice **s** and then is continued along a direction perpendicular to the one along which it was previously carried out, so that finally the incision is reversed "T"-shaped up to the urethral meatus **17**, wherein the metallic, bladder probe **E** is inserted.

The incision **r** is carried out in the conditions in which a Pean clamp **F** is fixed at the level of the uterine cervix **16**.

During the operating time II, by means of five Pean clamps **F** there takes place the removal, from the lateral side, of a vaginal mucosa **18** from the bladder **19** as well as of the latter from the anterior face of the uterine cervix **16** up to the level of an uterine isthmus **21.**

During the operating time III, the bladder-urethral junction **22** is identified by means of the index finger **23** of the right hand **G**, protected by a glove and at an angle of 45° formed by the longitudinal side on the urethral meatus **17** up to the posterior and horizontal vulvar commissure **24**, there takes place a paraurethral, retropubic and suprasymphyary penetration to carry out, in turn, one of the two right side and left side tunnels **t**, respectively. The right side tunnel **t** is made up to the level of a rectus abdominis muscles sheath. Then it is proceeded similarly for carrying aut left side tunnel **t**.

During the operating time IV, by means of the bistoury **D** the incision **r** is also continued circularly on the posterior face of the uterine cervix 16, then using the index finger **23** of the right hand G, the vaginal mucosa **18** is removed from the rectum.

During the operating time V there are performed the clamping, sectioning and ligating the cardical ligaments 24, preferably of 1 cm.

During the operting time VI, the isthmic strip **B** having the long portion **9** is fixed by suturing with the non-absorbable threads **12** on the lateral and posterior faces of the uterine isthmus **21**.

During the operating time VII, the short portion **10** of the isthmic strip B is sutured on the anterior face of the uterine isthmus **21** by means of the non-absorbable threads.

During the operating time VIII, there is performed, by means of the bistoury **D** a suprapubic incision u with a length of 4.0 ... 5.5 cm, up to the proximity of the rectus abdominis muscles sheath.

During the operating time IX, there is used the clamp **A** for passing the threads **11** and **15** and **12** and **14** for anchoring the uterine isthmus, in turn, through the orifices **f** and **e** through the tunnels **t**.

During the operating time **X**, the end zone **g** of the clamp **A** is introduced through the right side tunnel **t**, penetrating through the rectus abdominis muscles sheath, then the zone **g** is introduced through the left side channel **t** penetrating through the rectus abdominis muscles sheath.

During the operating time XI, there are carried out the anterior colpectomy and then the anterior colporrhaphy which consist in resecting, by means of the bistoury **D**, the vaginal mucosa **18** in excess on either side and in the anterior vaginal suture, followed by suturing the posterior incision suture on the uterine cervix **16**.

During the operating time XII, the ends of the non-absorbable threads **14** and **15** of the suburethral strip **C** which is mounted at the level of the cystourethral junction are anchored to the rectus abdominis muscles sheath, at the external extremities of the suprapubic incision **u**. these ends of the threads **14** and **15** are tied under the control of the graduated urethral probe **E** there being obtained al elongation of 1.5 cm of the urethra **25**.

During the operating time XIII, there are anchored to the rectus abdominis muscles sheath the ends of the threads **11** and **12** of the medial isthmic strip **B** as against the anchoring of the threads **14** and **15** of the suburethral strip **C**.

During the operating time XIV, there takes place the pulling and ligating the ends of the threads **11** and **12** of the isthmic strip **B** up to bringing the uterine cervix **16** in its intermediary anatomical position as close as possible to the normal position, without pulling it too high, so as not to open the urethro-rectal space in order to avoid elitrorectocele.

By means of the portion 10 the uterine isthmus **21** is covered and fixed on the anterior face in order to prevent slipping or tilting the same, and the end **o** of the portion **10** is cut according to the patient's dimensions
During the operating time XV, the suprapubic transverse incision u suture takes place.

During the operating time XVI, the posterior colpoperineorrhaphy with the myorrhaphy of the anus raising muscles takes place.

The method claimed by the invention may also be applied on an uterine cervix **16** with benign lesions or hypertrophic elongation of the uterine cervic **16**, and after fixing the isthmic strip **B** there takes place the amputation of the uterine cervix **16** by following the previously described operating times I...XVI.

The isthmic strip **B** which is made of a non-absorbable material constitutes a permanent implant, like a hammok having the uterine cervix **16** located thereon and which is fixed to the rectus abdominis muscles sheath by the ends of the threads **11** and 12, which make the recurrence not possible.

The clamp **A** is rigid, allowing to guide the blunt tip **d** which, in its turn, is smooth in order to avoid the vascular and visceral lesions, particularly those of urinary bladder. The curvature of zone **g** is so chosen as to be adapted to be introduced and extracted into/from the retropubic tunnels **t** and to allow keeping direct contact with the posterior face of the pubic symphysis and externalizing the blunt tip **d** above the upper edge of the pubic symplysis.

The short arms **7** and **8** on the left and right side, respectively, are positioned parallelly to the urethral meatus **17** and allow the clamp A orientation both towards the right side and towards the left side, as against the urethral meatus **17**, indicating the inclination angle of the clamp **A** corresponding to the angle of 45° of each of the retropubic tunnels **t**.

The additional ring **6** maintains the clamp **A** orientation, facilitating the perforation of the aponeurosis of the rectus abdominis muscles.

The presence of the orifices e and f in the zone 9 makes possible to pass the threads **11** and **15** and the threads **12** and **14** of the strips Band **C**, respectively.

By mounting the suburethral strip **C** at the level of the cystourethral junction the stress urinary incontinence is solved.

From the date of 25.10.2012 up to the, date of 30.06.2013 there were hospitalized 7 cases in the Clinical Hospital "Polizu", said cases after clinical and paraclinical investigations - "mictional cystography" - fractional curettage biopsy, resection with loop diathermy of the uterine cervix in order to exclude the associated pathology, there was diagnosed the uterus prolapse degree II-III, for which there was performed the uterine isthmus anchoring through the strip to the rectus abdormnis muscles sheath, as claimed by the invention.

The first patient, 59 years of age, came to the hospital for stress urine loss, heaviness in the lower portion of pelvis, introitus irritation and ulceration.

After performing the clinical and paraclinical investigations, there was diagnosed genital prolapse degree II, stress urinary incontinence, and lesion cervix.

The uterine isthmus was anchored to the rectus abdominis muscles sheath by means of strips B and C.

A peculiarity of this case is the presence of a uterine cervix lesion requiring the uterine cervix amputation.

Post-surgery, the patient resumed the normal physiological micturition 3 days after the removal of the endovesical probe, subsequently having the bladder residual of about 30 ml, i.e. within the normal values. The patient was externalized 6 days post-surgery with healed condition.

Eight months after the surgery the patient came for examination with good bladder retention, upon the genital examination with valves, the uterine cervix was in its anatomical position, upon manoevering the valves the patient does not lose urine.

In other 5 case it was proceeded as claimed by the invention, with the uterine cervix amputation due to the lesionss present thereon.

The seventh patient, 50 years of age, has as anatomo-clinical peculiarities a cystocele permagna, prolapse degree II with the following anatorno-pathological post fractional curettage biopsy and endocevical polyp resection result: "Endocervical fragments with polypoid aspect, endometrium fragments with simple endometrial hyperplasia".

On the date of 20.02.2013 it was proceeded with regard to anchoring, with the strip, the uterine isthmus to the rectus abdominis muscles sheath and urethrocystopexy.

In this case the uterine cervix amputation was not necessary, since the uterine cervix appears without pathological modifications demonstrated by Babes-Papanicolau colposcopy and cytopathology; but having an endometrial polyp for which a month before the polyp resection operation and fractional curettage biopsy were performed, the anatomo-pathological results showing the benignity thereof.

In all the previously mentioned cases there was performed the pre-surgery and post-surgery mictional cystography. The post-surgery mictional cystography showed the lower pole of the urinary bladder with irregular contour which is situated at the level of pubic symphysis, i.e. the ascent of this pole that was much previously lowered below the lower margin of pubic symphysis.

All the patients were externalized 6-7 days post surgery with good bladder relation and bladder residual in the range of 0-50 ml and the disappearance of all symptoms accused upon hospitalization, such as: stress urine loss, disappearance of -heaviness in the lower portion of pelvis, the sensation that "something falls out/sits on the ball", healing of intruitus irritation and vagina ulceration, disappearance of lumbar walking and urination pains.

In the patients that returned for examination after 4-5 months, the clinical examination by the use of the valves showed that the uterine cervix is situated in its normal position, while keeping the retention during the valve manoeuvering and the disappearaance of all symptoms they accused before the surgery.

These patient will be monitored for a period of minimum 1-3 years in order to demonstrate that the recurrence after the intervention as claimed by the invention does not exist, since the technique is based on a polypropylene material and non-absorbable threads, anchored to the rectus abdominis muscles sheath, which is a hormonally independent tissue which does not relax by aging.

## Claims

1. Medical instruments kit comprising a bistoury, two surgical scissors, six Pean clamps, three Kocher clamps, a metalic bladder probe, two Farabeuf spacing devices, a clamp (**A**) for anchoring the uterine isthmus and some isthmic and suburethral strips (**B** and **C**), respectively, the clamp (**A**) consisting of two long rigid arms (**1** and **2**) provided with some posterior portions (**a** and **b**) spaced apart from each other, joined at the lower side by means of a bridge (**3**), in front of which the distance between the arms (**1** and **2**) has the maximum value, the latter being non-removably joined together to form an anterior portion (**c**), tapered towards an anterior smooth blunt tip (**d**), in the immediate proximity thereof, in a proximal curved zone (**g**) of the anterior portion (**c**) there being cut some upper and lower orifices (e and f), respectively, in front of the bridge (**3**) the long arms (**1** and **2**) being distally provided with two left side and right side rings (**4** and **5**), respectively, an additional ring (**6**) being integrally attached to a lower portion (**i**) of the right side ring (**5**), to the outer side of the long arms (**1** and **2**), in the proximity of the bridge (**3**) and before the beginning of the anterior portion (**c**) there being located two short left side and right side arms (**7** and **8**), respectively, said arms ending with some smooth blunt tips (**g** and **h**).

2. Kit according to claim 1, **characterized in that** the clamp (**A**) for anchoring the uterine isthmus has a length of 28 cm, and at the level of the additional ring (**6**) the long arms (**1** and **2**) are rectilinear for a length of 22 cm and curved on a length of 6 cm, with an angle (α) of 30° in a proximal curved zone (**g**).

3. Kit according to claim 1, **characterized in that** the short arms (**7** and **8**) are located at a distance of 4 cm from the bridge (**3**) and have a length of 2.5 cm and form an angle *(β)* of 20° with the long arms (**1** and **2**).

4. Kit according to claims 1 and 2, **characterized in that** at a distance of 1.0 cm from the blunt tip (**d**) of the proximal curved zone (**9**) there are provided the anterior and posterior orifices (**e** and **f**), respectively, said orifices having a diamter of 4.5...5.5 mm.

5. Kit according to claim 1, **characterized in that** the isthmic strip (**B**) is made of a medically acceptable non-absorbable material, such as propylene and consists of two long and short portions (**9** and **10**), respectively, the short one being integrally attached to the long portion (**9**) in front of a medial zone (**l**) thereof, some ends (**m** and **n**) of the long portion (**9**) being linked by one of some threads (**11** and **12**) made of the same non-absorbable material, and an inner end (**o**) of the short portion (**10**) being free.

6. Kit according to claims 1 and 5, **characterized in that** the size of the meshes of the non-absorbable material that the long and short portions (**9** and **10**) are made up of is of 0.03 mm, the lengths of the portions (**9** and **10**) are of 16.0 cm and 8.0 cm, respectively, and their width is of 1.2 cm.

7. Kit according to claim 1, **characterized in that** the suburethral strip (**C**) consists of a body (**13**) made of polypropylene which has some ends (**p** and **q**), each tied with one of some threads (**14** and **15**) made of polypropylene, the length of body (**13**) being of 10.0 cmand the width thereof being of 1.2 cm.

## Patentansprüche

1. Medizinisches Instrumentarium, bestehend aus einem Klappmesser, zwei Scheren, sechs Pean-Klemmen, vier Kocher-Klemmen, einer Metallblasensonde, zwei Farabeuf-Abstandshaltern. Es zeichnet sich dadurch aus, dass es auch eine Klammer (A) zur Verankerung des Uterus-Isthmus sowie einige Isthmusstreifen und Suburethralstreifen (B und C) enthält, wobei die Klammer (A) aus zwei langen, starren Armen (1 und 2) besteht, versehen mit einigen hinteren Abschnitten (a und b), die voneinander getrennt sind und inferior durch eine Brücke (3) verbunden sind, durch die der Abstand zwischen den Armen (1 und 2) den Maximalwert hat. Wobei letzterer zwischen ihnen vereinigt ist, sie bilden unersetzlich einen vorderen Teil (c), der zu einer Spitze (d) schlank ist, die in unmittelbarer Nähe in einem gekrümmten proximalen Bereich (g) des zu übenden vorderen Teils (c) anterior glatt ist. Die oberen und unteren Löcher (e und f) sind in der Nähe der Brücke (3) und die langen Arme (1 und 2) sind distal versehen mit zwei Ringen (4 und 5) von links bzw. rechts, von einem unteren Teils (i) des Rings (5) rechts ist ein zusätzlicher Ring (6), der sich an der Außenseite der langen Arme (i und 2) in der Nähe der Brücke (3) und vor dem Beginn des vorhergehenden Teils (c) befindet, zwei kurze Arme (7 und 8) werden links und rechts platziert und mit einer der stumpfen, glatten Spitzen (g und h) abgeschlossen.

2. Medizinisches Instrumentarium nach Anspruch 1, **dadurch gekennzeichnet, dass** die Klammer (A) zur Verankerung des Uterus-Isthmus eine Länge von 28 cm aufweist und vom Ring (6) zusätzlich die langen Arme (1 und 2) mit einer Länge von 22 cm geradlinig sind und über eine Länge von 6 cm gekrümmt sind, mit einem Winkel von 30 ° in einem Bereich (g) gekrümmt, proximal.

3. Medizinisches Instrumentarium nach Anspruch 1, **dadurch gekennzeichnet, daß** die kurzen Arme (7 und 8) in einem Abstand von 4 cm von der Brücke (3) angeordnet sind, eine Länge von 2,5 cm haben und mit den Armen (1 und 2) lang ein Winkel von 20" ausmachen.

4. Medizinisches Instrumentarium nach den Ansprüchen 1 und 2, **dadurch gekennzeichnet, dass** in 1 cm Entfernung von der Spitze (d) des gekrümmten proximalen Bereichs (g) die Löcher vorgesehen sind und die Löcher (f) anterior bzw. posterior einen Durchmesser von 3 0 ... 4,0 mm aufweisen, zwischen denen ein Abstand von 4,5 ... 5,5 mm liegt.

5. Medizinisches Instrumentarium nach Anspruch 1, **dadurch gekennzeichnet, dass** der Isthmusstreifen (B) aus einem nicht resorbierbaren, medizinisch akzeptierten Material wie Propylen besteht und aus zwei langen und kurzen Teilen (9 bzw. 10) besteht. Letzterer ist solidarisch mit dem langen Abschnitt (9) in der Nähe seines Mittelbereichs (l). Einige Enden (m und n) des langen Abschnitts (9) sind mit einem von einigen Fäden (11 und 12) aus demselben nicht absorbierbaren Material verbunden, und ein inneres Ende (o) des kurzen Abschnitts (10) ist frei.

6. Medizinisches Instrumentarium nach den Ansprüchen 1 und 6, **dadurch gekennzeichnet, daß** die Maschenweite des nicht resorbierbaren Materials, aus dem die langen und kurzen Abschnitte (9 und 10) bestehen, 0,03 mm beträgt, die Längen der Abschnitte (9 und 10) betragen 16,0 cm und 8,0 cm, und ihre Breite beträgt 1,2 cm.

7. Medizinisches Instrumentarium nach Anspruch 1, **dadurch gekennzeichnet, dass** der suburethrale Streifen (C) aus einem Körper (13) aus Polypropylen besteht, dessen einige Enden (p und q) mit einem der aus (14 und 15) bestehenden Fäden aus Polypropylen verbunden sind, mit einer Körperlänge (13) von 10,0 cm und einer Breite von 1,2 cm.

## Revendications

1. Kit d'instruments médicaux, incluant un scalpel, deux ciseaux, six pinces Péan, quatre pinces Kocher, une sonde à vessie en métal, deux dépanneurs Farabeuf, **caractérisé en ce qu'**il contient aussi une pince (A) pour ancrer l'isthme utérin et des lanières (B et C) d isthme et sous-urétrale, respectivement, pince (A) étant composée de deux bras (1 et 2) longs, rigides, pourvus de quelques portions (a et b) postérieures, éloignés les uns des autres, unis, inférieur, par un pont (3), contre lequel la distance entre les bras (1 et 2) a la valeur maximale, les derniers étant unis, non détachables, formant une portion (c) antérieure, amincie à une extrémité (d) franche, lisse, antérieure, dans son voisinage immédiat, dans une zone (g) courbe, proximale à la portion (c) antérieure faisant quelques trous (e et f) haut et bas respectivement, près du pont (3) les bras (1 et 2) longs, étant prévues distal, avec deux anneaux (4 et 5) a gauche et droite respectivement, d'une portion (i) en bas de l'anneau (5) de la droite étant un anneau de solidarité (6) supplémentaire, a l'extérieur des bras (1 et 2) longs, près du pont (3) et avant le début de la portion (c) antérieure étant placés deux bras (7 et 8) courts, gauche et droite respectivement, aboutissant en des extrémités (g et h) franches, lisses.

2. Kit, selon la revendication 1, **caractérisé en ce que** la pince (A) pour ancrer l'isthme utérin, avec une longueur de 28 cm, et au niveau de l'anneau (6) supplémentaire les bras (1 et 2) longs sont rectilignes en longueur de 22 cm et courbés sur une longueur de 6 cm, avec un angle de 30° dans une zone (g) courbée, proximale.

3. Kit, selon la revendication 1, **caractérisé en ce que** les bras (7 et 8) courts ils sont placés à une distance de 4 cm du pont (3), ont une longueur de 2,5 cm et forment avec les bras (1 et 2) longs un angle (3 de 20°.

4. Kit, selon les revendications 1 et 2, **caractérisé en ce qu'**a une distance de 1,0 cm de l'extrémité (d) franche de la zone (g) courbée, proximale sont prévues les trous (e et f) antérieur et, respectivement, postérieur avec un diamètre de 3,0...4,0 mm, entre lesquels il y a une distance de 4,5...5,5 mm.

5. Kit, selon la revendication 1, **caractérisé en ce que** la bandelette (B) pour l'isthme est fabriquée d'un matériau non résorbable, médicalement accepté, comme la propylène et se compose de deux parties (9 et 10) longues et, respectivement, courte, ce dernier étant solidaire de la partie (9) longue à côté d'une zone (l) médiane, des extrémités (m et n) de la portion (9) longue étant liées à l'un des fils (11 et 12) faits du même matériau non résorbable, et une extrémité (o) intérieure de la portion (10) courte étant libre.

6. Kit, selon les revendications 1 et 6, **caractérisé en ce que** la taille des mailles du matériau non résorbable duquel sont fabriquées les portions (9 et 10) longue et courte est de 0,03 mm, les longueurs des portions (9 et 10) sont de 16,0 cm et respectivement, 8,0 cm, et leur largeur est de 1,2 cm.

7. Kit, selon la revendication, **caractérisé en ce que** la bandelette (C) sous urétrale se compose d'un corps (13) en polypropylène, avec des extrémités (p et q) liées à l'un des fils (14 et 15) fabriquées en polypropylène, la longueur du corps (13) étant de 10,0 cm, et son largeur de 1,2 cm.
